# EUROPÄISCHE PATENTSCHRIFT

(11) **EP 2 804 668 B1**
(45) Veröffentlichungstag und Bekanntmachung des Hinweises auf die Patenterteilung: **28.06.2017**
(21) Anmeldenummer: 13700644.1
(22) Anmeldetag: 10.01.2013
(51) Int. Cl.: A61N 5/06, A61N 1/36

(54) **VORRICHTUNG ZUR NEUROSTIMULATION**
DEVICE FOR NEUROSTIMULATION
DISPOSITIF DE NEUROSTIMULATION

(30) Priorität: 19.01.2012 DE 202012000492 U; 26.03.2012 DE 202012003100 U
(43) Veröffentlichungstag der Anmeldung: 26.11.2014
(73) Patentinhaber: EBS Technologies GmbH, 14532 Kleinmachnow (DE)
(72) Erfinder: WARSCHEWSKE, Udo, 12249 Berlin (DE)
(74) Vertreter: Kruspig, Volkmar
(86) Internationale Anmeldenummer: PCT/EP2013/050335
(87) Internationale Veröffentlichungsnummer: WO 2013/107678

(56) Entgegenhaltungen:
- EP-A1- 0 412 629
- WO-A1-2010/140073
- DE-A1-102004 048 982
- DE-U1-202011 050 692
- US-A- 3 612 061
- US-A- 3 865 099
- US-A1- 2004 176 820
- US-A1- 2006 079 946
- US-A1- 2007 185 541

## Beschreibung

Die Erfindung betrifft eine Vorrichtung zur Neurostimulation, umfassend eine die Augenpartie des Probanden bedeckende Maske, Brille oder dergleichen Augenvorsatz, welcher gegenüber der Umgebung optisch dicht ausgebildet ist und zur Augenseite gerichtet jeweils eine Leuchtfläche aufweist, die mit einer Licht im sichtbaren Spektralbereich emittierenden Quelle in Verbindung steht oder eine solche Lichtquelle enthält, sowie mit Mitteln zum Fixieren am Kopf des Probanden gemäß Anspruch 1.

Aus der DE 20 2011 050 692 U1 ist eine Vorrichtung zur Lichtstimulation von biologischem Gewebe, insbesondere für den Augenbereich vorbekannt. Diese Vorrichtung umfasst wenigstens ein Mittel, welches einen bewegbaren Lichtpunkt erzeugt und weiterhin eine Atemfrequenz für den Verwender vorgibt. Darüber hinaus sind weitere Mittel vorhanden, welche Lichtwellen des Nahen infraroten Spektrums auf das Gewebe des Verwenders zur Einwirkung bringen.

Die vorbekannte Lösung soll eine Stimulation eines Probanden auf der Basis der Verbesserung des Herz-Kreislauf-Zustands ermöglichen und darüber hinaus den Gesundheitszustand des Auges in Verbindung mit einer Kräftigung der Augenmuskulator ermöglichen.

Die notwendigen Leuchtmittel der Vorrichtung sind jeweils einem oder beiden Augen zugeordnet und es sind die Lichtquellen für den Nahen infraroten Spektralbereich im Bereich der Glabella und/oder im Schläfenbereich und/oder im Occipitalbereich in die Vorrichtung integriert. Der Strahlungswinkel bezogen auf die emittierte infrarote Strahlung soll sehr gering sein, um eine große Eindringtiefe zu bewirken.

Konkret wird die Vorrichtung als Lichtbrille, d.h. rahmenartig oder displayartig ausgestaltet. In der Mitte des Displays ist eine Position für ein weiteres Leuchtmittel oder eine Gruppe von weiteren Leuchtmitteln befindlich, vor denen sich eine Streuscheibe befindet. Eine Vielzahl von ersten Leuchtmitteln ist um die Streuscheibe verteilt angeordnet. Die Ansteuerung der Leuchtmittel erfolgt über einen Mikrokontroller, wobei bevorzugt die Leuchtmittel Bewegungsmuster erzeugen, z.B. in Form des Eindrucks eines Kreises oder einer Schleife des sich bewegenden Lichtpunkts.

Bei der Lern-Entspannungsvorrichtung zur Stimulation des Zentralnervensystems und der Gehirnströme eines Menschen nach DE 38 23 402 A1 erfolgt eine Übertragung pulsierender Lichtsignale auf das linke und das rechte Auge sowie pulsierender Tonsignale auf das linke und das rechte Ohr unter Ausschaltung sonstiger optischer und akustischer Wahrnehmungen aus der Umgebung.

Mit dieser Vorrichtung soll eine Eigenbehandlung möglich werden. Im linken und rechten Brillenglas sind zum Zweck der optischen Stimulation Leuchtdioden angeordnet, welche getrennt voneinander ansteuerbar sind. Dabei sollen sich die Leuchtdioden waagerecht mittig auf einem abgedunkelten Brillenglas bzw. einer undurchsichtigen Fläche befinden, die wiederum vom Brillengestell aufgenommen wird. Weiterhin ist eine das Brillengestell umfassende Abdeckung vorhanden, die seitlich eintretendes Licht abschirmt und damit das Blickfeld des Benutzers auf die Leuchtdioden beschränkt.

Die DE 10 2008 012 669 A1 offenbart eine Vorrichtung und ein Verfahren zur visuellen Stimulation mit einer Mehrzahl von Stimulationselementen, die visuelle Stimulationssignale erzeugen, welche bei einer Aufnahme über ein Auge eines Patienten und Weiterleitung an Neuronen, die eine krankhaft synchrone und oszillatorische neuronale Aktivität aufweisen, die Phase der neuronalen Aktivität der Neuronen zurücksetzen. Die Steuereinheit betreibt die Stimulationseinheit derart, dass mindestens zwei vorgesehener Stimulationselemente die visuellen Stimulationssignale zeitversetzt zueinander und/oder mit unterschiedlicher Phase und/oder unterschiedlicher Polarität erzeugen.

Bei einer der Ausführungsformen nach DE 10 2008 012 669 A1 ist neben einer Transmissionsbrille die Anordnung epikutaner, d.h. auf der Haut des Patienten befestigbarer EEG-Elektroden erläutert. Die dortige Steuereinheit verstärkt die mittels der EEG-Elektroden gemessene Potentialdifferenz und verwendet diese nach entsprechender Verrechnung zur Ansteuerung der transmissionsmodulierten Gläser der Transmissionsbrille.

Die Transmissionsbrille besteht aus zwei Einfassungsteilen mit je einem transmissionsmodulierten Brillenglas, Ohr-Bügeln, mit welchen die Brille hinter dem Ohr des Patienten mechanisch gehalten wird, und entsprechender Steuereinheit, die die Transmission der transmissionsmodulierten Gläser der Brille ändert. Alternativ wird auch auf die Verwendung von partiell durchsichtigen oder undurchsichtigen Lichtbrillen als Stimulationsbrille verwiesen.

Bei der Vorrichtung zur Elektrostimulation des Auges gemäß DE 10 2010 027 201 A1 umfasst diese ein brillenartiges Gestell, das ein Nasenteil und eine mit dem Nasenteil verbundene Anordnung zum Halten des Gestells am Kopf des Patienten aufweist.

An dem Nasenteil ist zumindest eine Stimulationselektrode angeordnet. Weiterhin sind am Nasenteil zwei Elektrodenhalter vorgesehen, zwischen denen eine auswechselbare, drahtförmige Stimulationselektrode eingespannt werden kann.

Es liegt also nach diesem Stand der Technik ein maskenartiges Gebilde vor, das im Bereich der Stirn, der Wangen und der Nase flächig auf dem Gesicht des Patienten aufliegt, wobei für das Auge eine angepasste Augenöffnung vorgesehen ist. Die betreffende Vorrichtung ermöglicht durch die Elektrodenausbildung einen guten, reproduzierbaren Kontakt zwischen der jeweiligen Stimulationselektrode und dem zu behandelnden Auge und kann von einem blinden oder nahezu blinden Patienten problemlos benutzt werden.

Bekannt sind darüber hinaus brillenartige Vorrichtungen mit einem die Augen abdeckenden Teil, an dem mehrere Elektroden angeordnet sind. Konkret sind z.B. nach der US 2004/0176820 Vorrichtungen bekannt, bei denen um die Augenpartie herum mehrere Elektroden vorgesehen sind, die punktförmig Kontakt zu dem die Augen unmittelbar umgebenden Gewebe herstellen, wobei diese Elektroden mit einem Stimulationsgerät verbunden werden, welches Signale erzeugt, die als Stimulationssignale über die Elektroden in die Augen geführt werden. Eine entsprechende Gegenelektrode ist an beliebiger Stelle des Körpers des Patienten angebracht.

Die gattungsbildende DE 10 2004 048 982 A1 zeigt einen Augenvorsatz für ein Stimulationsgerät zum Stimulieren des Zentralnervensystems und der Gehirnströme des Menschen. Der Augenvorsatz ist so ausgestaltet, dass er, vor den Augen getragen, die Augen gegen Lichteinfall von außen abschirmt. Weiterhin ist mindestens eine Lichtquelle zum Abgeben variierender Lichtsignale vorgesehen und es ist die mindestens eine Lichtquelle derart am Augenvorsatz angeordnet oder in diesen integriert, dass ihr Licht die Augen nicht direkt erreicht.

Auch ist wenigstens ein von der mindestens einen Lichtquelle beleuchtetes Element derart in den Augenvorsatz integriert oder an diesem befindlich, dass das Licht der Lichtquelle die Augen über dieses beleuchtete Element indirekt erreicht. Bei Verwenden der Brille nach DE 10 2004 048 982 A1 treten keine unangenehm empfundenen hohen Lichtintensitäten auf.

Bei einer Ausführungsform kann das beleuchtete Element flächig ausgebildet werden. Die Größe der Fläche und ihre Anordnung im Augenvorsatz bzw. ihre Integration in den Augenvorsatz können derart aufeinander abgestimmt sein, dass das Sehfeld eines Auges oder beider Augen durch die Fläche weitgehend ausgefüllt wird.

Bei einer Ausgestaltung ist die Lichtquelle derart an mindestens einer die Vorderseite mit der Rückseite verbindenden Seitenfläche einer transparenten Platte angeordnet, dass das Licht zwischen Vorder- und Rückseite in die Platte hinein abgestrahlt wird. Das zwischen die Vorder- und Rückseite der Platte eingestrahlte Licht wird in der Platte gestreut und gelangt so indirekt in das Auge bzw. in die Augen des Trägers des Augenvorsatzes. Insbesondere dann, wenn die mindestens eine Lichtquelle an der oberen Seitenfläche der transparenten Platte angeordnet wird, entsteht der Eindruck eines natürlichen Lichteinfalls von oben.

Aus den Patentanmeldungen EP-A1-0,412,629 und A1 und WO-A1-2010/140073 sind auch Augenvorsätze zur Neurostimulation einer Augenpartie umfassend Leuchtquellen und Elektroden vorbekannt.

Aus dem Vorgenannten ist es daher Aufgabe der Erfindung, eine weiterentwickelte Vorrichtung zur Neurostimulation, umfassend eine die Augenpartie des Probanden bedeckende Maske, Brille oder dergleichen Augenvorsatz, welcher gegenüber der Umgebung optisch dicht ausgebildet ist und zur Augenseite gerichtet jeweils eine Leuchtfläche aufweist, anzugeben, die in einfacher Weise herstell- und handhabbar ist und welche den hygienischen Anforderungen bei einer Anwendung sowohl im klinischen als auch privaten Bereich genügt.

Die Lösung der Aufgabe der Erfindung erfolgt durch eine Vorrichtung zur Neurostimulation gemäß der Merkmalskombination nach Anspruch 1, wobei die Unteransprüche mindestens zweckmäßige Ausgestaltungen und Weiterbildungen darstellen.

Es wird demnach von einer Vorrichtung zur Neurostimulation, umfassend eine die Augenpartie des Probanden bedeckende Maske, Brille oder dergleichen Augenvorsatz ausgegangen, welcher gegenüber der Umgebung optisch dicht ausgebildet ist und zur Augenseite des Probanden gerichtet jeweils eine Leuchtfläche aufweist, die mit einer Licht im sichtbaren Spektralbereich emittierenden Quelle in Verbindung steht oder eine solche Lichtquelle enthält, sowie mit Mitteln zum Fixieren am Kopf des Probanden, z.B. in Form von Brillenbügeln oder einem elastischen Band. Erfindungsgemäß ist ein einseitig offener Grundkörper, z.B. gefertigt aus einem Kunststoffmaterial, vorgesehen, welcher in seinem Inneren die jeweilige Leuchtfläche und Lichtquelle aufnimmt. Diesbezüglich besitzt der Innenkörper einen Hohlraum, auch um eine Berührung mit den Augenlidern beim Tragen des Augenvorsatzes auszuschließen.

Am Grundkörper sind oberhalb und/oder unterhalb des die Augenpartie abdeckenden Bereichs Elektrodenaufnahmen oder Elektrodenabstützflächen vorgesehen.

Weiterhin ist am Grundkörper ein flexibler, austauschbarer kissen- oder mattenartiger Flächenkörper fixierbar, welcher zu den Elektrodenaufnahmen oder Elektrodenabstützflächen komplementäre Durchgangsöffnungen besitzt, wobei der Flächenkörper im Gebrauch der Vorrichtung am Augenpartie-Stirnbereich des Probanden zur Anlage kommt und die Elektroden epikutan ausgebildet sind.

Der Flächenkörper kann nach Benutzung leicht vom Grundkörper getrennt und insbesondere unter hygienischem Aspekt entsorgt oder aber je nach Art des Flächenmaterials desinfiziert und einer Wiederverwendung zugeführt werden. Der kissen- oder mattenartige Flächenkörper bewirkt durch seine stofflichen Eigenschaften ein angenehmes Tragegefühl und behindert einen seitlichen Lichteintritt zwischen Grundkörper und der Haut des Probanden. Gemäß der Erfindung sind die Elektroden zweiteilig ausgebildet. Beide Elektrodenteile können über eine Snap-in-Verbindung miteinander verbunden und gleichzeitig am Flächenkörper befestigt werden.

Die Elektroden können somit vom Flächenkörper getrennt werden, so dass eine Wiederverwendung der Elektroden grundsätzlich möglich ist. Durch die Durchgangsöffnungen im Flächenkörper und die Verbindung des Flächenkörpers mit dem Grundkörper ist eine jederzeit reproduzierbare Lage der Elektroden gewährleistet.

In weiterer Ausgestaltung der Erfindung kann am zur Augen- oder Stirnpartie weisenden Elektrodenteil eine Kontaktkappe oder eine Feuchte aufnehmende Kappe bzw. ein dementsprechender Überzug angeordnet werden.

Die Kontaktkappe kann bevorzugt aus einem Edelmetall, z.B. Gold bestehen oder eine derartige Beschichtung aufweisen, um den Übergangswiderstand Elektrodenoberfläche / Hautoberfläche zu minimieren und für eine entsprechende widerstandsseitige Reproduzierbarkeit zu sorgen.

Die Feuchte aufnehmende Kappe oder der Feuchte aufnehmende Überzug kann aus einem Schaumstoff-, Filz- oder Vliesmaterial bestehen. Durch gezieltes Befeuchten, auch z.B. mit einer Kochsalzlösung, kann der Kontaktwiderstand minimiert werden.

Der vorbeschriebene Flächenkörper kann aus einem Schaumgummimaterial, einem Abstandsgewirke oder dergleichen Material gefertigt sein.

Die vorerwähnten Elektroden sind grundsätzlich symmetrisch verteilt angeordnet. Bei einer bevorzugten Ausführungsform sind vier Elektroden vorhanden, wobei jeweils am linken und rechten Auge oberhalb und unterhalb des Auges die Elektroden im Grundkörper bzw. im Flächenkörper befindlich sind.

Die Leuchtfläche kann bei einer Ausführungsform der Erfindung als Streuscheibe ausgeführt sein, wobei über mindestens eine der jeweiligen Stirn- oder Seitenflächen Strahlung eingekoppelt wird.

Die Streuscheibe(n) sind am Grundkörper von der offenen Seite her einsetz- und austauschbar ausgeführt.

Die Leuchtfläche kann aber auch Bestandteil eines Miniatur-Monitors bzw. eines Miniatur-Displays sein, um die gewünschte optische Anregung des Probanden über die Retina zu gewährleisten.

Der Grundkörper besteht bevorzugt aus einem nachgiebigen Material oder weist mindestens im Bereich, der am oder oberhalb des Nasenbeins zur Anlage kommt, einen flexiblen, gelenkartigen Abschnitt auf, um ein bequemes Tragen und eine ausreichende Anlage am Kopf des Probanden je nach den anatomischen Gegebenheiten sicherzustellen.

Am Grundkörper sind weiterhin elektrische Anschlussmittel, z.B. ausgeführt als Stecker oder Buchse, insbesondere in koaxialer Ausführung, vorhanden.

Weiterhin ist mindestens eine Referenz- oder Gegenelektrode am Hals- oder Schulterbereich des Probanden angeordnet.

Die mindestens eine Referenz- oder Gegenelektrode kann dann mit dem betreffenden Anschlussmittel am Grundkörper kontaktiert werden, wobei die erfindungsgemäße Vorrichtung mit einem Steuergerät in Verbindung steht, welches Stimulationssignale zur optischen und elektrischen Neurostimulation zur Verfügung stellt. Als ein solches Stimulationsgerät kann eine Lösung zum Einsatz kommen, die in der DE 20 2006 021 009 offenbart ist. Der Gegenstand dieser Offenbarung wird hiermit vollinhaltlich zum Bestandteil der vorliegenden Beschreibung gemacht.

Die Erfindung soll nachstehend anhand von Ausführungsbeispielen sowie unter Zuhilfenahme von Prinzipskizzen näher erläutert werden.

Hierbei zeigen:
- Fig. 1: eine Darstellung einer ersten Ausführungsform der erfindungsgemäßen Vorrichtung zur Neurostimulation, ausgebildet als brillenartige Maske, am Kopf eines Probanden befindlich;
- Fig. 2: eine Darstellung der erfindungsgemäßen Vorrichtung mit erkennbarem Flächenkörper, Elektroden und Leuchtflächen, d.h. mit Blick in die Innenseite des Grundkörpers ohne Haltebügel oder Halteband;
- Fig. 3: eine Darstellung der Vorrichtung mit weggenommenem Grundkörper und der vom Probanden abgewandten Seite des Flächenkörpers mit erkennbaren Aktivelektroden im Augen- bzw. Stirnbereich sowie der Referenz- oder Gegenelektrode auf der Schulter;
- Fig. 4: eine Explosivdarstellung wesentlicher Komponenten der erfindungsgemäßen Vorrichtung mit Detailabbildung des Aufbaus der am Flächenkörper befindlichen Aktivelektroden und
- Fig. 5: eine Explosivdarstellung einer zweiten Ausführungsform der erfindungsgemäßen Vorrichtung.

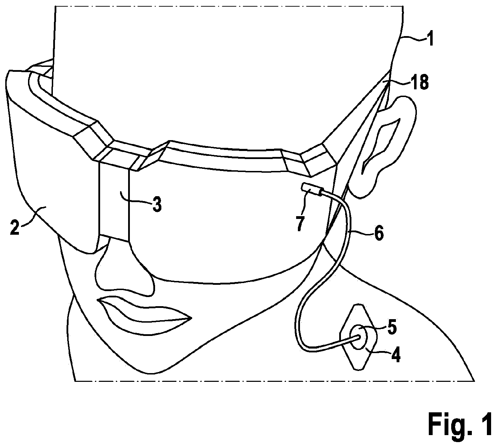

Gemäß Fig. 1 ist am Kopf eines Probanden 1 die erfindungsgemäße Vorrichtung zur Neurostimulation befestigt.

Die Fig. 1 lässt den Grundkörper 2 erkennen, der so ausgebildet ist, dass ein Augenvorsatz entsteht, der blickdicht den Bereich der Augen des Probanden 1 abdeckt.

Der Grundkörper 2 kann hierbei zweigeteilt als linksseitige und rechtsseitige Augenabdeckung mit elastischem Gelenk 3, die Teile verbindend, ausgeführt werden.

Eine Referenzelektrode 4 ist beim gezeigten Beispiel im linken Schulterbereich des Probanden 1 fixiert, wobei die Referenzelektrode 4 einen Kontakt 5 umfasst, um über ein Kabel 6 eine elektrische Verbindung zu einem weiteren Kontaktanschluss 7 am Grundkörper 2 herzustellen.

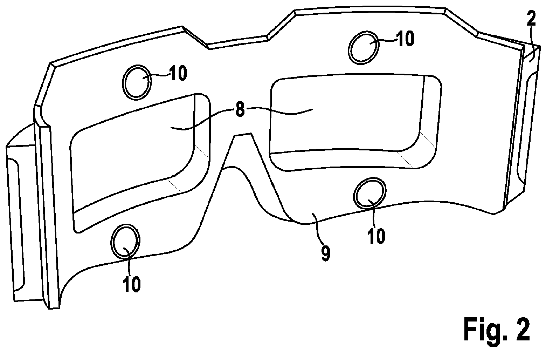

Bei der Darstellung nach Fig. 2 ist quasi die Innen- oder Rückseite des Grundkörpers 2 zu erkennen.

In den Hohlräumen des Grundkörpers 2 befindet sich eine Leuchtquelle für das linke und rechte Auge, z.B. ausgebildet als Leuchtfläche 8. Die Leuchtflächen 8 können auch als LED- oder LCD-Monitore realisiert werden.

Die zum Probanden 1 weisende Seite des Grundkörpers 2 ist von einem Flächenkörper 9 abgedeckt, der kissen- oder mattenartig ausgeführt wird. Der Flächenkörper 9 sorgt für ein angenehmes Tragegefühl und kann aus hygienischen Gründen leicht ausgewechselt und damit ausgetauscht werden.

Durch Öffnungen im Flächenkörper 9 ragen Elektroden 10, jeweils angeordnet oberhalb und unterhalb der Augenpartie bzw. der Leuchtflächen 8.

Die epikutan ausgebildeten Elektroden 10 können eine Außenoberfläche aus einem Edelmetall, z.B. Gold besitzen, um den Kontaktübergangswiderstand zur Haut des Probanden zu minimieren bzw. reproduzierbar zu gestalten.

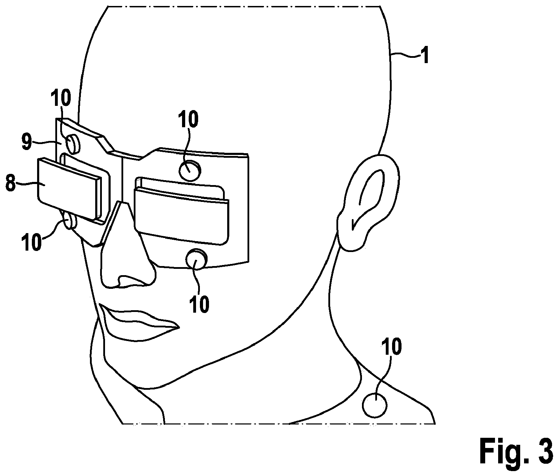

Um die Anordnung des Flächenkörpers 9 mit Elektroden 10 zu verdeutlichen, sei auf die Fig. 3 aufmerksam gemacht, welche die Vorrichtung mit weggebrochenem Grundkörper zeigt.

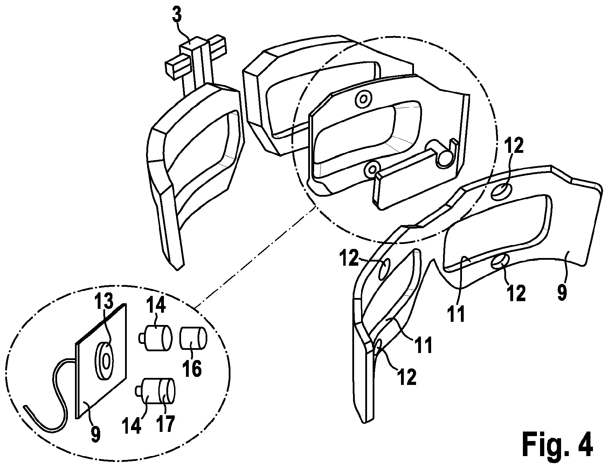

Bei der Explosivdarstellung und der Detaildarstellung des Aufbaus der Elektroden nach Fig. 4 wird der Sandwichaufbau der Vorrichtung ersichtlich.

Im Flächenkörper 9 sind Aussparungen 11 für einen freien Blick auf die Leuchtflächen 8 sowie die bereits erwähnten Durchgangsöffnungen 12 für den Durchtritt der Elektroden 10 vorhanden.

Die Elektroden 10 bestehen aus zwei Teilen, nämlich einem ersten Teil 13 und einem zweiten Teil 14.

Das erste Teil 13 und das zweite Teil 14 können durch eine Snap-in-Mechanik verbunden werden.

Diesbezüglich können die Elektrodenhälften 13 und 14 unter Zwischenschaltung eines entsprechenden Abschnitts des Flächenkörpers 9 an selbigem fixiert werden.

Zum Erhalt reproduzierbarer Eigenschaften besteht weiterhin die Möglichkeit, die Elektrodenhälfte oder das Elektrodenteil 14, welches in Richtung zur Hautoberfläche des Probanden 1 orientiert ist, mit einem Filz 16 oder einer Edelmetallkontaktoberfläche 17 zu versehen.

Die als Kappe ausführbare Filzanordnung 16 kann mit einer Flüssigkeit getränkt werden, um den Kontaktübergangswiderstand zur Hautoberfläche zu verringern.

Die Elektroden, insbesondere das Elektrodenteil 13, kann in einer Elektrodenaufnahme am Grundkörper 2 (die Aufnahme ist in den Figuren nicht gezeigt) geführt oder fixiert werden. Alternativ kann eine entsprechende Fläche am Grundkörper auch lediglich zur Abstützung der entsprechenden Elektrode dienen, wobei das in der Fig. 1 ersichtliche Band 18 für einen festen Sitz der erfindungsgemäßen Vorrichtung am Kopf des Probanden 1 sorgt.

Anstelle der in der Fig. 4 offenbarten Snap-in-Verbindung der Elektrodenteile oder Elektrodenhälften 13, 14 kann auch eine Schraubverbindung oder eine bajonettähnliche Befestigungsart gewählt werden.

Die der Haut des Probanden abgewandte Seite des Flächenkörpers kann über eine Adhäsionsbeschichtung verfügen, um für eine temporäre Fixierung des Flächenkörpers am Grundkörper zu sorgen.

Besteht der Flächenkörper 9 beispielsweise aus einem Abstandsgewirke, besteht die Möglichkeit, leitfähige Polfäden vorzusehen, um eine elektrische Kontaktierung der Elektroden zu erreichen. Darüber hinaus ist das Abstandsgewirke Feuchte aufnehmend, d.h. hautoberflächlicher Schweiß des Probanden wird aufgenommen und abgeführt, was den Tragekomfort der erfindungsgemäßen Vorrichtung weiter verbessert.

Bei einer diesbezüglichen Ausgestaltung des Flächenkörpers 9 können in diesen Elektrodenoberflächen eingewirkt oder in sonstiger Weise eingebettet werden, wobei dann lediglich rückseitig für eine Kontaktverbindung zu Gegenkontaktelementen am oder im Grundkörper Sorge getragen wird.

Der Flächenkörper kann leicht vom Grundkörper getrennt und unter hygienischen Aspekten entsorgt oder bei geeigneter Materialauswahl einem Desinfektionsschritt unterzogen und wiederverwendet werden.

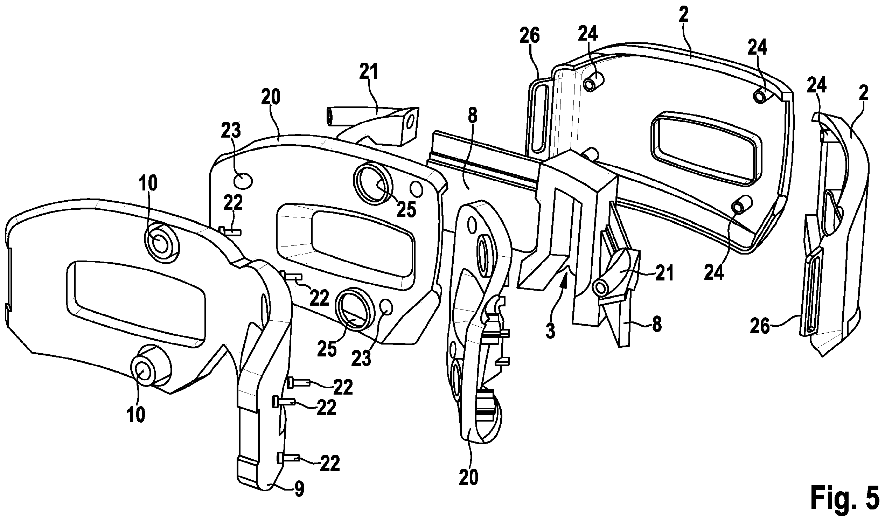

Bei dem Ausführungsbeispiel der erfindungsgemäßen Vorrichtung nach Fig. 5 wird der Grundkörper durch zwei Außenschalen 2 sowie zwei Innenschalen 20 gebildet.

Zwischen den Außenschalen 2 und den Innenschalen 20 ist ein Trägerteil mit Leuchtflächen 8 befindlich. Das Trägerteil, welches im gezeigten Beispiel aus zwei Hälften besteht, ist über einen elastischen Nasenbügel 3 verbunden. Weiterhin sind Zuleitungen aufnehmende Knickschutzelemente 21 am Trägerteil mit den Leuchtflächen befestigbar.

Über Schraubverbindungen 22 sowie Durchgangsbohrungen 23 in den Innenschalen erfolgt eine Befestigung unter Nutzung zapfenförmiger Vorsprünge 24 an den Außenschalen 2 unter gleichzeitiger Fixierung der Leuchtflächen 8.

Der Flächenkörper 9 ist bei der Ausführungsform gemäß Fig. 5 als Schaumstoffpolster ausgebildet und weist Ausnehmungen für die Elektroden 10 auf. Die Elektroden 10 können an den Innenschalen und dort vorgesehenen Rastelementen 25 über eine Snap-in-Verbindung fixiert werden.

An den Außenschalen 2 sind seitliche Fortsätze 26 vorhanden, die der Aufnahme eines Halterungs- bzw. Kopfbands dienen.

Es sei an dieser Stelle noch darauf aufmerksam gemacht, dass prinzipiell per elektronischer Schaltung jede der Elektroden permanent oder kurzzeitig als Referenzelektrode fungieren kann. Ein Anschluss 7 gemäß dem ersten Ausführungsbeispiel ist nur dann erforderlich, wenn die Referenzelektrode an einer Stelle außerhalb der erfindungsgemäßen Vorrichtung, z.B. im Schulterbereich, befestigt werden soll.

Erfindungsgemäß besteht grundsätzlich die Möglichkeit, die Vorrichtung nicht nur mittels eines Haltebands (siehe Fig. 1, Bezugszeichen 18) am Kopf eines Probanden zu befestigen. Vielmehr kann die brillenartige Vorrichtung auch mit einer EEG-Kappe gekoppelt werden, wobei die EEG-Kappe am Kopf, beispielsweise mittels Kinn- oder Schulterriemen, verspannt wird.

Neben der Möglichkeit, die Elektroden zu befeuchten, ist weiterhin vorgesehen, Elektroden so auszubilden, dass diese Ausformungen aufweisen, in die ein Kontaktmittel, z.B. ein elektrisch leitendes Gel, eingebracht werden kann.

## Patentansprüche

1. Vorrichtung zur Neurostimulation, umfassend eine die Augenpartie des Probanden (1) bedeckende Maske, Brille oder dergleichen Augenvorsatz (9), welcher gegenüber der Umgebung optisch dicht ausgebildet ist und zur Augenseite gerichtet jeweils eine Leuchtfläche (8) aufweist, die mit einer Licht im sichtbaren Spektralbereich emittierenden Quelle in Verbindung steht oder eine solche Lichtquelle enthält, sowie mit Mitteln (18) zum Fixieren am Kopf (1) des Probanden,
wobei
ein einseitig offener Grundkörper (2) vorgesehen ist, welcher in seinem Inneren die jeweilige Leuchtfläche (8) und Lichtquelle aufnimmt, wobei am Grundkörper (2) oberhalb und/oder unterhalb des die Augenpartie abdeckenden Bereichs Elektrodenaufnahmen oder Elektrodenabstützflächen, in denen Elektroden (10, 13, 14) angeordnet sind, vorgesehen sind,
weiterhin am Grundkörper (2) ein flexibler, austauschbarer kissen- oder mattenartiger Flächenkörper (9) fixiert ist, welcher zu den Elektrodenaufnahmen oder Elektrodenabstützflächen komplementäre Durchgangsöffnungen (12) besitzt, wobei der Flächenkörper (9) im Gebrauch der Vorrichtung am Augenpartie-Stirnbereich des Probanden (1) zur Anlage kommt und die Elektroden (10) epikutan ausgebildet sind,
wobei
die Elektroden (10, 13, 14) zweiteilig ausgebildet und beide Elektrodenteile (13, 14) über eine Snap-in-Verbindung am Flächenkörper (9) befestig- und verbindbar sind.

2. Vorrichtung nach Anspruch 1,
**dadurch gekennzeichnet, dass**
am zur Augen- oder Stirnpartie weisenden Elektrodenteil (13, 14)eine Kontaktkappe (16, 17) oder eine Feuchte aufnehmende Kappe (16, 17) befindlich ist.

3. Vorrichtung nach Anspruch 2,
**dadurch gekennzeichnet, dass**
die Kontaktkappe (17) aus Edelmetall besteht oder eine derartige Beschichtung aufweist.

4. Vorrichtung nach Anspruch 2,
**dadurch gekennzeichnet, dass**
die Feuchte aufnehmende Kappe (16)aus einem Schaumstoff-, Filz- oder Vliesmaterial besteht.

5. Vorrichtung nach einem der vorangegangenen Ansprüche,
**dadurch gekennzeichnet, dass**
der Flächenkörper (9) aus einem Schaumgummimaterial, Abstandsgewirke oder dergleichen Material besteht.

6. Vorrichtung nach einem der vorangegangenen Ansprüche,
**dadurch gekennzeichnet, dass**
die Elektroden (10) symmetrisch verteilt angeordnet sind.

7. Vorrichtung nach einem der vorangegangenen Ansprüche,
**dadurch gekennzeichnet, dass**
die Leuchtfläche (8) als Streuscheibe ausgebildet ist, wobei über mindestens eine der jeweiligen Stirn- oder Seitenflächen Strahlung eingekoppelt wird.

8. Vorrichtung nach Anspruch 7,
**dadurch gekennzeichnet, dass**
die Streuscheibe(n) am Grundkörper (2) von der offenen Seite her einsetz- und austauschbar ausgebildet sind.

9. Vorrichtung nach einem der Ansprüche 1 bis 6,
**dadurch gekennzeichnet, dass**
die Leuchtfläche (8) ein Miniatur-Monitor oder ein Miniatur-Display ist.

10. Vorrichtung nach einem der vorangegangenen Ansprüche,
**dadurch gekennzeichnet, dass**
der Grundkörper (2) aus einem nachgiebigen Material besteht und/oder der Bereich, der am oder oberhalb des Nasenbeins zur Anlage kommt, einen flexiblen, gelenkartigen Abschnitt (3) aufweist.

11. Vorrichtung nach einem der vorangegangenen Ansprüche,
**dadurch gekennzeichnet, dass**
am Grundkörper (2) elektrische Anschlussmittel (7), insbesondere ausgeführt als Stecker oder Buchse, vorhanden sind.

12. Vorrichtung nach einem der vorangegangenen Ansprüche, insbesondere nach Anspruch 11,
**dadurch gekennzeichnet, dass**
mindestens eine Referenz- oder Gegenelektrode (4), die insbesondere mit dem Anschlussmittel am Grundkörper (2) kontaktiert ist, im Hals-Schulterbereich des Probanden (1) vorgesehen ist.

13. Vorrichtung nach Anspruch 1,
**dadurch gekennzeichnet, dass**
die Elektroden (10) eine rücksprungartige Ausformung aufweisen, welche der Aufnahme eines elektrisch wirksamen Kontaktmittels dienen.

14. Vorrichtung nach einem der vorangegangenen Ansprüche,
**dadurch gekennzeichnet, dass**
der Grundkörper (2) mehrteilig ausgebildet ist und Außenschalen (2) sowie Innenschalen (20) aufweist, wobei zwischen den Außenschalen (2) und den Innenschalen (20) Leuchtflächen (8) angeordnet sind.

## Claims

1. A device for neurostimulation, comprising a mask, glasses or similar eye attachment (9) covering a subject's (1) eye area, which attachment is formed to be optically impervious relative to the environment and exhibits, in each case directed toward the eye side, a luminous surface (8) being in communication with a source emitting light in the visible spectral range or including such a light source, as well as means (18) for fixing to the subject's (1) head,
wherein
a base body (2) that is open on one side is provided which accommodates the respective luminous surface (8) and light source in its interior, wherein on the base body (2) above and/or below the section covering the eye area electrode receptacles or electrode supporting surfaces are provided in which electrodes (10, 13, 14) are arranged,
furthermore, a flexible, exchangeable pad-like or mat-like sheet (9) having passage openings (12) that are complementary to the electrode receptacles or electrode supporting surfaces is fixed to the base body (2), wherein, during use of the device, the sheet (9) gets to rest against the subject's (1) eye area/front area, and the electrodes (10) are configured to be epicutaneous,
wherein
the electrodes (10, 13, 14) are configured to be two-part, and both electrode parts (13, 14) are attachable and connectable to the sheet (9) via a snap-in connection.

2. The device according to claim 1,
**characterized in that**
on the electrode part (13, 14) pointing toward the eye area or front area, a contact cap (16, 17) or a humidity absorbing cap (16, 17) is located.

3. The device according to claim 2,
**characterized in that**
the contact cap (17) is made of noble metal or comprises a coating of this type.

4. The device according to claim 2,
**characterized in that**
the humidity absorbing cap (16) is made of a foam, felt or non-woven material.

5. The device according to any one of the preceding claims,
**characterized in that**
the sheet (9) is made of a foamed rubber material, spacer fabric or similar material.

6. The device according to any one of the preceding claims,
**characterized in that**
the electrodes (10) are arranged to be symmetrically distributed.

7. The device according to any one of the preceding claims,
**characterized in that**
the luminous surface (8) is formed as a diffusing disk, wherein radiation is fed in via at least one of the respective front or side surfaces.

8. The device according to claim 7,
**characterized in that**
the diffusing disk(s) is/are configured to be insertable and exchangeable on the base body (2) from the open side.

9. The device according to any one of the claims 1 to 6,
**characterized in that**
the luminous surface (8) is a miniature monitor or a miniature display.

10. The device according to any one of the preceding claims,
**characterized in that**
the base body (2) is made of a resilient material, and/or the area getting to rest against or above the nasal bone exhibits a flexible, articulation-like portion (3).

11. The device according to any one of the preceding claims,
**characterized in that**
electrical connection means (7), in particular configured as a connector or socket, are present on the base body (2).

12. The device according to any one of the preceding claims,
in particular according to claim 11,
**characterized in that**
at least one reference or counter electrode (4) which is in particular contacted to the connection means on the base body (2) is provided in the subject's (1) neck/shoulder area.

13. The device according to claim 1,
**characterized in that**
the electrodes (10) exhibit a recess-like shaping serving the purpose of receiving an electrically active contact agent.

14. The device according to any one of the preceding claims,
**characterized in that**
the base body (2) is configured to be multi-part and exhibits outer shells (2) and inner shells (20), wherein luminous surfaces (8) are arranged between the outer shells (2) and inner shells (20).

## Revendications

1. Dispositif de neurostimulation, comportant un masque, un organe formant lunettes ou un recouvrement oculaire similaire (9) recouvrant la zone des yeux du participant (1), qui est réalisé de façon optiquement opaque par rapport à l'environnement et qui présente une surface lumineuse (8) dirigée vers le côté des yeux, qui est en communication avec une source émettant de la lumière dans la plage spectrale visible ou bien qui contient une telle source de lumière, ainsi que des moyens (18) pour la fixation sur la tête (1) du participant,
dans lequel
il est prévu un corps de base (2) ouvert sur un côté qui reçoit dans son volume intérieur la surface lumineuse respective (8) et la source de lumière,
et dans lequel il est prévu sur le corps de base (2), au-dessus et/ou au-dessous de la zone recouvrant la zone des yeux, des logements d'électrode ou des surfaces de support d'électrode, dans lesquel(le)s sont agencées des électrodes (10, 13, 14),
et de plus un corps surfacique (9) flexible, interchangeable, en forme de coussin ou de matte est fixé sur le corps de base (2), le corps surfacique possédant des ouvertures traversantes (12) complémentaires aux logements d'électrode ou aux surfaces de support d'électrode,
dans lequel, pendant l'utilisation du dispositif, le corps surfacique (9) vient en appui contre la zone des yeux et du front du participant (1), et les électrodes (10) sont réalisées de façon épicutanée,
et dans lequel les électrodes (10, 13, 14) sont réalisées en deux parties et les deux parties d'électrode (13, 14) sont susceptibles d'être fixées et reliées sur le corps surfacique (9) par une liaison à encliquetage.

2. Dispositif selon la revendication 1,
**caractérisé en ce que**
sur la partie d'électrode (13, 14) dirigée vers la zone des yeux ou du front, il est prévu un capuchon de contact (16, 17) ou un capuchon (16, 17) absorbant de l'humidité.

3. Dispositif selon la revendication 2,
**caractérisé en ce que**
le capuchon de contact (17) est constitué en métal noble ou comprend un tel revêtement.

4. Dispositif selon la revendication 2,
**caractérisé en ce que**
le capuchon (16) absorbant de l'humidité est constitué en un matériau en mousse, en feutre ou en non-tissé.

5. Dispositif selon l'une des revendications précédentes,
**caractérisé en ce que**
le corps surfacique (9) est constitué en un matériau en caoutchouc mousse, en un tricot d'écartement ou en un matériau similaire.

6. Dispositif selon l'une des revendications précédentes,
**caractérisé en ce que**
les électrodes (10) sont agencées en répartition symétrique.

7. Dispositif selon l'une des revendications précédentes,
**caractérisé en ce que**
la surface lumineuse (8) est réalisée sous forme de disque de diffusion, le rayonnement étant injecté par l'une au moins des surfaces frontales ou latérales respectives.

8. Dispositif selon la revendication 7,
**caractérisé en ce que**
le(s) disque(s) de diffusion sont réalisés de façon insérables et interchangeables depuis le côté ouvert sur le corps de base (2).

9. Dispositif selon l'une des revendications 1 à 6,
**caractérisé en ce que**
la surface lumineuse (8) est un moniteur miniature ou un affichage miniature.

10. Dispositif selon l'une des revendications précédentes,
**caractérisé en ce que**
le corps de base (2) est constitué d'un matériau souple et/ou la zone qui vient en appui sur ou au-dessus de l'os nasal comprend une portion flexible (3) semblable à une articulation.

11. Dispositif selon l'une des revendications précédentes,
**caractérisé en ce que**
sur le corps de base (2) sont prévus des moyens de connexion électriques (7), réalisés en particulier sous forme de fiche mâle ou femelle.

12. Dispositif selon l'une des revendications précédentes, en particulier selon la revendication 11,
**caractérisé en ce que**
dans la zone du cou et de l'épaule du participant (1), il est prévu au moins une électrode de référence ou électrode antagoniste (4) qui est mise en contact en particulier avec le moyen de connexion sur le corps de base (2).

13. Dispositif selon la revendication 1,
**caractérisé en ce que**
les électrodes (10) présente une conformation en forme de retrait qui sert à recevoir un moyen de contact à effet électrique.

14. Dispositif selon l'une des revendications précédentes,
**caractérisé en ce que**
le corps de base (2) est réalisé en plusieurs parties et comprend des coques extérieures (2) ainsi que des coques intérieures (20), des surfaces lumineuses (8) étant agencées entre les coques extérieures (2) et les coques intérieures (20).
